Europäisches Patentamt

European Patent Office

Office européen des brevets

(19)

(11) Publication number: **0 186 502**
**A2**

# EUROPEAN PATENT APPLICATION

(12)

(21) Application number: **85309438.1**

(22) Date of filing: **23.12.85**

(51) Int. Cl.⁴: **A 61 J 3/07**

(30) Priority: **25.12.84 IL 73925
17.12.85 IL 77360**

(43) Date of publication of application:
**02.07.86 Bulletin 86/27**

(84) Designated Contracting States:
**AT BE CH DE FR GB IT LI LU NL SE**

(71) Applicant: **Tyroler, Jano**

**Kfar Szold(IL)**

(72) Inventor: **Tyroler, Jano**

**Kfar Szold(IL)**

(74) Representative: **Adams, William Gordon et al,
RAWORTH, MOSS & COOK 36 Sydenham Road
Croydon Surrey CR0 2EF(GB)**

(54) **Flavor capsule and method of preparing same.**

(57) A controlled release flavor capsule comprising a thin walled capsule made of non-rubbery, resilient, water insoluble, non-toxic plastic, and filling material containing extractable flavors and/or juices enclosed in said capsule, said capsule defining one or more small slits which open when pressure is applied to the capsule, said capsule being of suitable dimensions to be completely concealable inside the mouth.

*Fig . 1a.*

*Fig . 1b.*

FLAVOR CAPSULE AND METHOD OF PREPARING SAME          0186502

## FIELD OF THE INVENTION

The present invention relates to controlled release flavour capsules, particularly to capsules intended for chewing and/or sucking, thereby extracting flavours and/or juices contained therein. Most particularly, the invention is concerned with tobacco capsules which release tobacco flavour and/or juices in a controlled manner and are thus helpful in enabling persons to stop smoking or at least to reduce their desire and need to smoke as well as continue to enjoy tobacco.

## BACKGROUND OF THE INVENTION

Smoking cigarettes presents a serious health hazard. This is due, in great measure, to the burning of tobacco which generates tars, carbon monoxide and other undesirable products which enter the lungs during smoking. The intake of tobacco juice, particularly nicotine, does, however, give pleasure to the smoker and it would be desirable to be able to provide means for delivering to the mouth the tobacco juices but not the undesirable combustion products of smoking.

To this end several solutions have been proposed. Among these U.S. Patent No. 3,877,468 discloses a chewing gum containing tobacco alkaloid. U.S. Patent No. 3,757,798 describes a pouch made of tea-bag paper filled with tobacco. This pouch is to be placed in the mouth so that saliva mixes with the tobacco inside the pouch dissolving some of the juice and then passes back through the pouch into the mouth. This pouch, which is made of paper is not readily chewable since it is likely to be perforated when bitten and release some tobacco, therefore restricting the user to gentle sucking action only.

Food capsules are also known. Thus U.S. Patent No. 3,620,759 describes perforated gelatine capsules containing soluble food extracts which are extracted in contact with water and are subsequently dissolved together with the capsule. The capsules were not suggested for use in the mouth.

## SUMMARY OF THE INVENTION

We have discovered that it is possible to provide a flavour capsule which will dispense flavours and/or juices such as, for example, tobacco flavour and/or juices, slowly and in a controlled manner by chewing and sucking. It is therefore the objective of this invention to provide a flavour capsule for controllably releasing liquid extractable flavours and/or juices contained therein by the application of pressure on the walls of the capsule.

Another objective is to provide a method for manufacturing such flavour capsules.

Yet another objective is to provide enjoyment of tobacco as well as a method to reduce dependency on cigarettes by substituting the chewing and/or sucking of capsules containing tobacco for actual smoking.

There is thus provided in accordance with the present invention a controlled release flavor capsule including a thin walled capsule made of non-rubbery, resilient, water insoluble, non-toxic plastic, and filling material containing extractable flavors and/or juices enclosed in the capsule, the capsule defining one or more small slits which open when pressure is applied to the capsule, the capsule being of suitable dimensions to be completely concealable inside the mouth.

According to one embodiment of the invention, the flavor capsule has a cylindrical shape and dimensions of about 10 to 25 mm long and 5 to 25 mm in diameter. According to an alternate embodiment, the flavor capsule has the shape of a round disc and is 10 to 30 mm in diameter and about 5 to 15 mm in height.

There is further provided in accordance with the present invention a process for manufacturing flavor capsules including filling hollow cartridges or tube sections of non-rubbery resilient, water-insoluble, non-toxic plastic with a filling material including extractable flavours and/or juices, sealing the cartridges or tube sections and providing at least one slit extending at least substantially through a wall of the cartridge or tube.

According to a preferred embodiment, the filling material comprises moist tobacco snuff.

Further according to a preferred embodiment, the step of filling includes inserting a filling sleeve into the open end of the cartridge, introducing moist fibrous filling material through the filling sleeve into the cartridge, removing the filling sleeve, compacting the filling material inside the cartridge to a level below the cartridge height, and sealing the cartridge above the level of the filling material.

Apparatus for filling flavour capsules according to the present invention is also provided.

## BRIEF DESCRIPTION OF THE DRAWINGS

The present invention will be further understood and appreciated from the following detailed description taken in conjunction with the drawings in which:

Figs. 1a and 1b show one embodiment of a cylindrical capsule constructed and operative in accordance with the present invention;

Figs. 2a and 2b represent another embodiment of a cylindrical capsule;

Figs. 3a and 3b show yet another type of cylindrical capsule;

Figs. 4a and 4b represent a capsule in the form of a disc-shaped tablet;

Fig. 5 illustrates an embodiment of apparatus for producing capsules according to the present invention; and

Figs. 6a and 6b illustrate detail views on the filling device of the apparatus of Fig. 5.

## DETAILED DESCRIPTION OF THE INVENTION

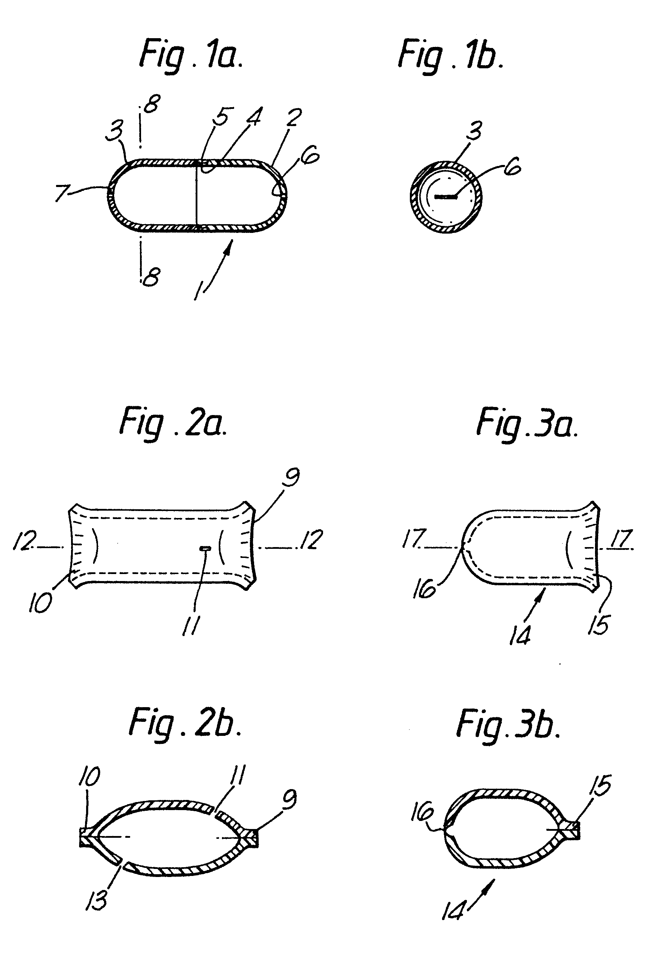

With reference to Fig. 1a, there is shown a side view of a cylindrical capsule 1 comprising matching body parts 2 and 3 sealed together at their overlapping points 4 and 5. Each body part has a slit 6,7 through its wall section.

Fig. 1b is a cross-section of Fig.1a taken at 8-8 showing a frontal view of the capsule with the slit 6 at its end. This shaped capsule could also comprise a cylindrical cap and a matching telescopically engaged body part similar in shape to the well-known glycerine capsules used for holding pharmaceutical preparations.

Fig. 2a shows a top view of a capsule comprising a tube section sealed at both ends 9 and 10. Slit 11 (a second slit preferably exists on the bottom side) enables the contents to be extracted upon applying pressure to the capsule as, for instance, by chewing and sucking the capsule.

Fig. 2b shows a frontal view of this capsule taken across 12-12 having slits 11 and 13. The ends 9, 10 of the tube are sealed.

0186502

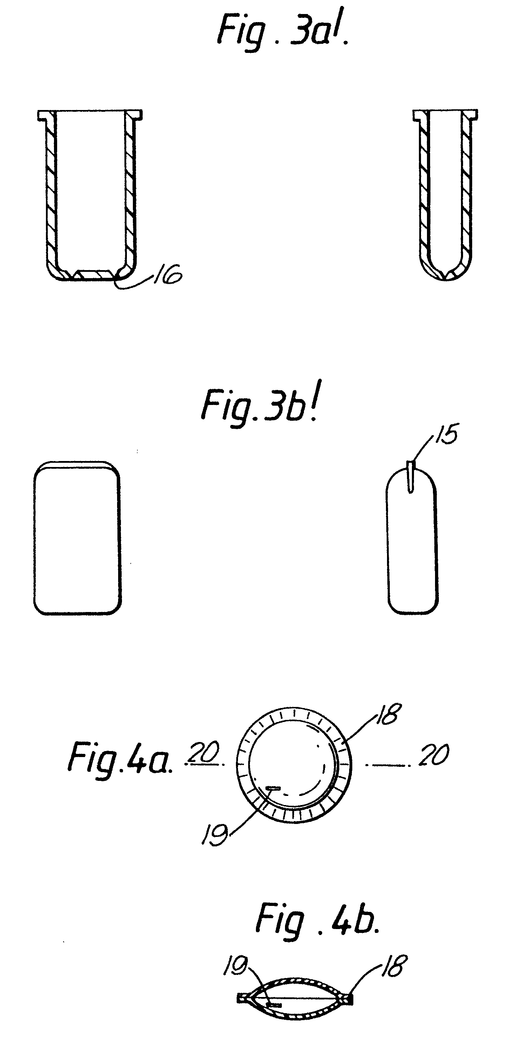

Fig. 3a is a top view of a plastic cylindrical cap 14 sealed at one end 15 and having only one slit 16. Fig. 3b shows this cap from a frontal view across 17-17.

Fig. 4a shows a top view of a disc-shaped capsule with a sealed plastic edge around its circumference 18 and slit 19. A second slit (not shown) is on the underside thereof. Fig.4b is a side view of this same capsule shown across 20-20.

The dimensions of the capsules are preferably about 10-25 mm in length and about 5-25 mm in diameter for cylindrical shapes, and about 10-30 mm in diameter and 5-15 mm high for disc shapes. The wall thickness of the capsules should range somewhere between 0.3 mm and 1.0 mm and preferably between 0.4 mm to 0.8 mm. It is a particular feature of the present invention that the capsules are of such a size that they are completely concealable within the mouth of the user.

The slits may be essentially round or elongate. According to a preferred embodiment, the length of the slits is equal to or larger than the capsule wall thickness. The size and shape of the slit need only be sufficient to allow passage of fluid through them when the capsules' physical shape is temporarily deformed by pressing, chewing or squeezing, but not allowing passage of the filling material, i.e., finely chopped tobacco, through the slits.

It will be appreciated that each of the slits in the capsules of the present invention may comprise an aperture penetrating the entire wall of the capsule, as shown in Fig. 1a. Alternatively, the slits may extend only substantially through the capsule wall, leaving a thin outer covering which is punctured upon application of pressure to the capsule, as illustrated in Figs. 3a and 3b. This latter embodiment of the slits permits the provision of an unbroken exterior capsule surface until the capsule is in use, thus preventing undesired drying, leakage or ingress of matter to the capsule. Furthermore, as stated above, the slits may be of any desired shape to permit selectable opening upon application of pressure to the capsule.

The capsules described above may be filled with flavour and/or juice containing material, sealed, and then slit. Alternatively, pre-slit plastic sleeves or tubes can be filled with flavour and/or juice containing material and sealed afterwards. Suitable flavour or juice containing material includes foods such as coffee, tea and spices; sweets, mouthwashes or breath fresheners, or stimulants, among other things. The flavour capsules of this invention have a particular utility as capsules for enclosing tobacco,

particularly moist snuff, and other fibrous, shredded materials, which may be moist or dry.

The capsules according to the present invention preferably are made of rigid, resilient, water-insoluble, non-toxic plastic, having sufficient resilience to withstand chewing without being perforated by the teeth. It is important that the capsule should not crack nor break and only the slit should enable the entry and exit of flavour with the help of mouth fluids such as saliva. The capsules should also have some springiness in order to give the person who is chewing the feeling that he is not merely biting a flat plastic disc.

Suitable plastics for this purpose are polyolefins, preferably polyethylene and polypropylene and co-polymers of these. Fluorocarbons, PVC, ionomers, nylons, polyester and other thermoplastic resins can also be used as long as they are safe for use in the mouth as approved by the proper health authorities.

Satisfaction by the user is achieved by placing the capsule entirely in the mouth and extracting the flavour and/or juice from the capsule by chewing between the teeth, pressing between tongue and palate or between the outer gums and lips or between lips and teeth, combined with sucking action. The extracted flavour and/or juice is diluted with small amounts of saliva and absorbed by the mouth tissues and digestive system.

Since the plastic capsule is resilient, it returns to its approximate original shape after deformation by chewing or squeezing. This elastic action creates a relative vacuum inside the capsule which draws in fresh saliva from the mouth.

One use for which the capsules of the present invention are particularly suited is for tobacco capsules. In these capsules, the original moisture of the tobacco is gradually extracted, fresh saliva enters through the slits into the capsule and dissolves more moisture and tobacco juice until all solubles are completely extracted from the tobacco. The rate of juice extraction is controlled by the force of the squeezing/sucking action and by the frequency thereof. The contents of one capsule containing about 0.5 to 1.0 gram of tobacco can be extracted by the average user within 25 minutes. Fresh capsules, naturally, require only very gentle squeezing to extract the desirable amount of juice. To maintain a constant rate of satisfaction, and particularly as the flavour content of the capsule nears depletion, chewing will become more vigorous.

- o -

The tobacco capsules of this invention, when substituted for cigarettes, have a number of advantages. Firstly, they satisfy a need of the smoker for nicotine while at the same time avoiding tar deposits in the lungs and inhalation of carbon monoxide. Moreoever, they eliminate throat and bronchial irritation, avoid staining of teeth by smoke, and are acceptable socially. Furthermore, the chewing and sucking action of the capsule satisfies the need of an individual to occupy his mouth.

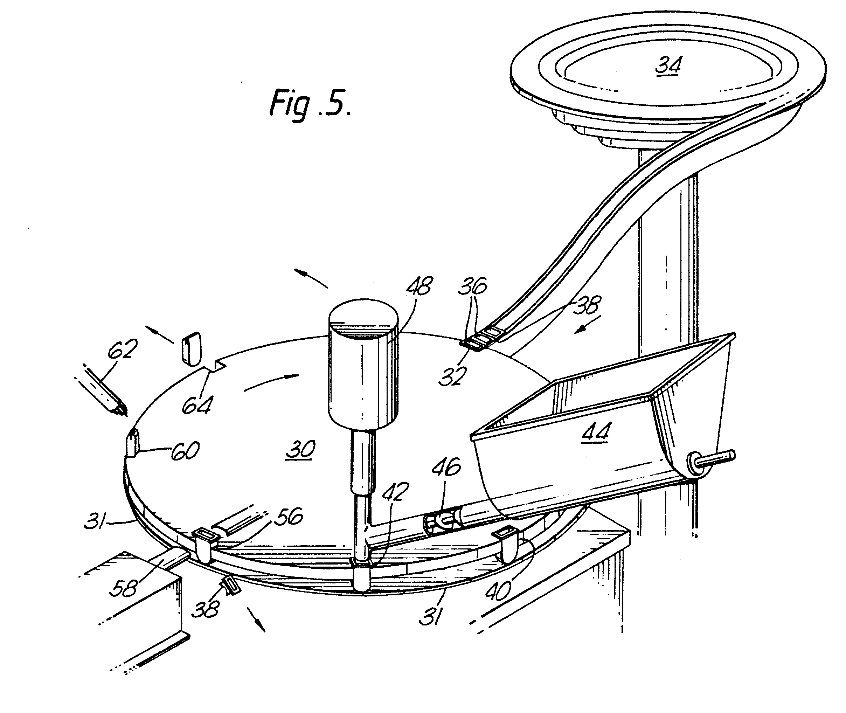

Referring now to Fig. 5 there is shown one embodiment of apparatus for producing flavour capsules according to the present invention. The apparatus comprises a rotating frame 30 coupled for rotation to a motor (not shown) and comprising a plurality of work stations, at each of which a different operation is performed. Affixed beneath rotating frame 30 is a stationary plate 31. The distance between plate 31 and frame 30 around the periphery of frame 30 differs at various locations therealong, as will be further explained hereinbelow.

The first work station 32 is coupled to a capsule feeding means 34 which may comprise a vibrating feeder. Capsule feeding means 34 provides a uniform flow of empty capsules 36 to frame 30. Each of capsules 36 preferably comprises a preformed cartridge sealed at one end thereof, preferably pre-slit, defining an upper outwardly extending flange 38. Capsule 36 depends from flange 38 in each of the work stations about rotating frame 30.

After one capsule 36 has entered work station 32, the frame rotates through sufficient arc to advance each capsule from one station to the next. The new capsule now reaches second work station 40 where sensor means (not shown), such as an electronic sensor, checks to insure that a capsule is, indeed, present. The capsule is then sterilized and the air inside removed by a flash of nitrogen gas, as known in the art.

At the next work station 42, the capsule is filled. The filling material is provided in a container 44 including a rotary screw or auger type feeder 46. Feeder 46 serves both to measure the desired dose or amount of material for each capsule and to carry it from container 44 to the filling apparatus generally designated 48.

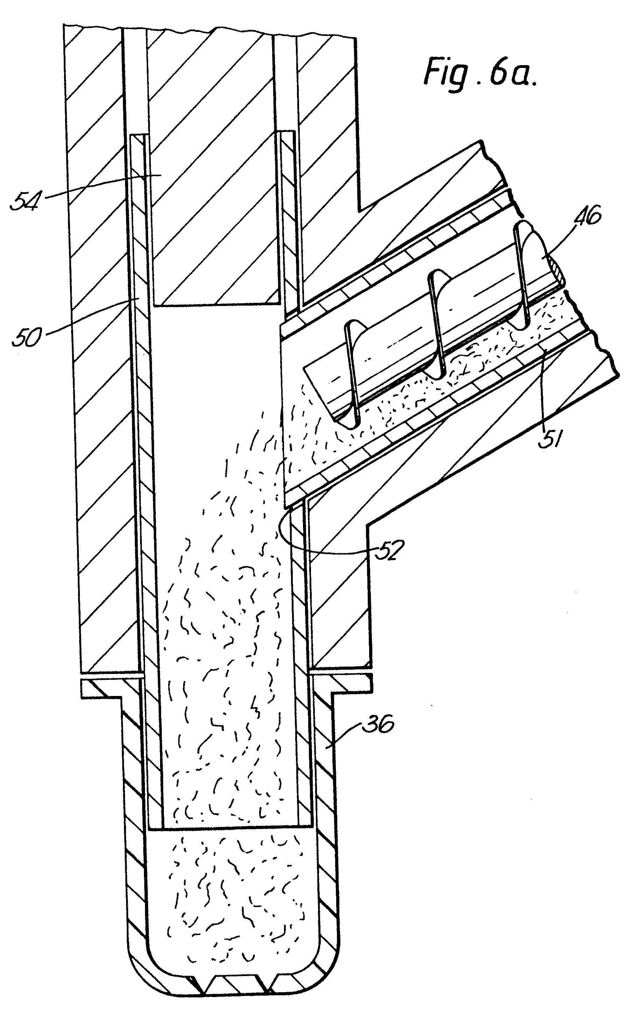

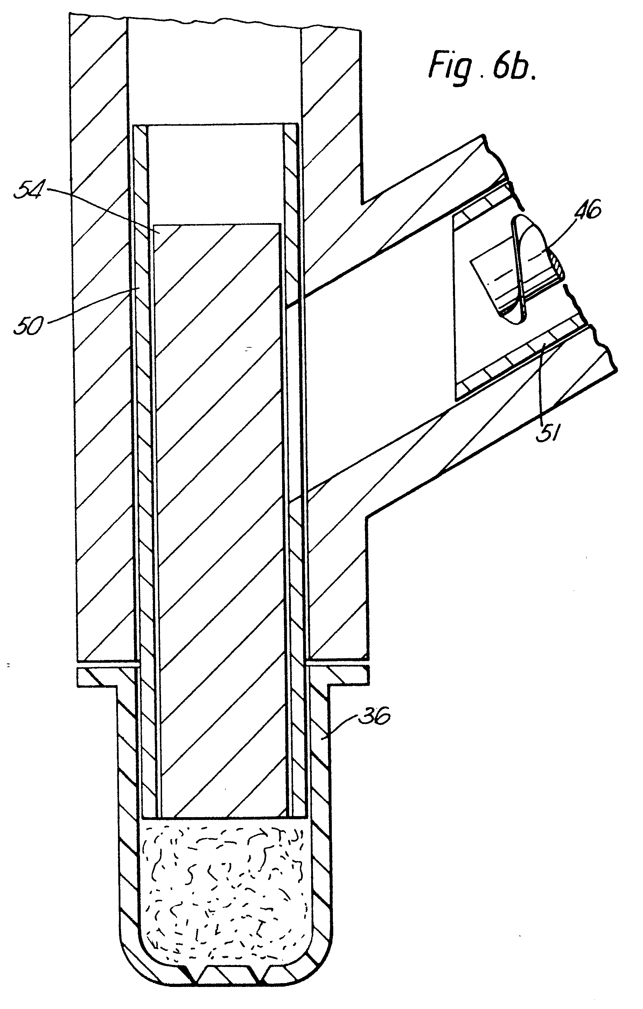

Filling apparatus 48 is shown in cut-away detail in Figs. 6a and 6b. Capsule 36 moves into work station 42 and a filling sleeve 50 slideably mounted within filling apparatus 48 slides into the capsule as the filling apparatus moves adjacent the open top of the capsule.

0186502

It is a particular feature of this embodiment of the invention that the filling sleeve 50 extends from within filling apparatus 48 to within capsule 36. This serves two purposes, first, to prevent leakage of moisture onto the flange or upper portion of the capsule, and second, to permit the introduction of loose filling material to an initial height greater than that of the capsule.

Sleeve 51 of screw feeder 46 is moved towards filling sleeve 50 and through an aperture 52 in the side thereof. A predetermined amount of material is introduced by feeder 46 through filling sleeve 50 into capsule 36. A slight vibratory movement may be generated in the feeder 46 to spill any loose material from the end of the feeder sleeve 51 into the capsule. Sleeve 51 is then retracted from aperture 52.

In the case where the filling material comprises tobacco or other fibrous material, it is desired to compress the material into the capsule. Accordingly, a piston or other means 54 reciprocatably mounted within filling apparatus 48 is extended through filling sleeve 50 compressing the material within capsule 36 to a height below the flange 38. Piston 54 then retracts, filling sleeve 50 is removed from capsule 36, and the frame rotates the filled capsule to the next work station 56.

Between work station 42 and work station 56, plate 31 defines an incline whereby the distance between plate 31 and frame 30 grows gradually smaller. As capsule 36 rotates towards work station 56, its bottom engages the inclined portion of plate 31 and the capsule is gradually raised within the slot in which is rests in frame 30. At work station 56, the top edges of the capsule are sealed to one another, preferably under a nitrogen flash, by any suitable means, such as ultrasonic sealing, and the upper flange is removed from the capsule by any known cutting means, such as opposing blades. The sealed and cut capsule then moves to work station 60 where it is flamed by a torch 62 to smoothen any sharp edges. The sealed capsule now progresses to the final station 64, where it is discharged from frame 30 by any suitable means.

It will be appreciated by those skilled in the art that additional work stations may be added as desired to frame 30 in order to carry out additional operations on the capsule. For example, if one is filling capsules with a dry material to which one wishes to add moisture, an additional work station including means for introducing moisture into the filling material within the filled capsule may be provided.

It will be appreciated by those skilled in the art that the invention is not limited to what has been described hereinabove by way of example. Rather, the scope of the invention is limited solely by the claims which follow.

CLAIMS:

1.   A controlled release flavor capsule comprising a thin walled capsule made of non-rubbery, resilient, water insoluble, non-toxic plastic, and filling material containing extractable flavors and/or juices enclosed in said capsule, said capsule defining one or more small slits which open when pressure is applied to the capsule, said capsule being of suitable dimensions to be completely concealable inside the mouth.

2.   A flavor capsule of claim 1 having acylindrical shape and dimensions of about 10 to 25 mm long and 5 to 25 mm in diameter.

3.   A flavor capsule of claim 1 having the shape of a round disc and being 10 to 30 mm in diameter and about 5 to 15 mm in height.

4.   A flavor capsule of claims 1 to 3 wherein the plastic capsule has a wall thickness of about 0.3 to 1.0 mm.

5.   A flavor capsule of claims 1 to 4 wherein the plastic capsule has slits ranging from 0.3 to 2.0 mm in length.

6.   A flavor capsule of claims 1 to 5 wherein the plastic capsule is made of polyethylene or polypropylene or co-polymers of these.

7.   A flavor capsule of claims 1 to 6 wherein the filling material is selected from coffee, tea, spices, mouthwash, or breath freshener, or mixtures of these.

8.   A flavor capsule of claims 1 to 6 wherein said filling material comprises tobacco.

9.   A flavor capsule according to any of claims 1 to 8 and wherein the plastic capsule has slits extending substantially through the capsule wall, said slits being arranged to open into apertures through the capsule wall upon application of pressure to the capsule.

0186502

10. A process for manufacturing flavor capsules of claims 1 to 9 comprising filling hollow cartridges or tube sections of non-rubbery resilient, water-insoluble, non-toxic plastic with a filling material comprising extractable flavors and/or juices, sealing the cartridges or tube sections and providing at least one slit extending at least substantially through a wall of said cartridge or tube.

11. A process for manufacturing flavor capsules of claims 1 to 9 comprising providing at least one suitable slit in a hollow, non-rubbery resilient, water-insoluble, non-toxic, plastic cartridge or tubular section, filling said section with filling material comprising extractable flavors and/or juices, and sealing said cartridge or tubular section.

12. A process as in claims 10 and 11 wherein said filling material comprises moist tobacco snuff.

13. A method for extracting flavors and/or juices from a flavor capsule of claims 1 to 8 comprising placing said flavor capsule in the mouth and extracting the flavors and/or juices by squeezing the capsule between the teeth or pressing it between the tongue and palate or between inner cheek or lips and outer gums and/or sucking the capsule.

14. A process according to any of claims 10 to 12 and wherein said step of filling comprises the steps of:
    inserting a filling sleeve into the open end of said cartridge;
    introducing moist fibrous filling material through said filling sleeve into said cartridge;
    compacting said filling material inside the cartridge to a level below the cartridge height;
    removing said filling sleeve; and
    sealing the cartridge above the level of the filling material.

15. Apparatus for filling flavor capsules according to claims 1 to 9 comprising:

a rotating frame defining a plurality of work stations;
means for introducing an empty cartridge to one of said
work stations on said frame;
means for filling said cartridge with a filling material
comprising:
a reciprocating filling sleeve coupled to said frame and
arranged to enter into said cartridge to be filled; and
means for sealing said filled cartridge.

16.     Apparatus according to claim 15 and further comprising:
a filling material feeder means and reciprocating feeder
sleeve coupled to said filling sleeve and arranged to
provide filling material in predetermined quantities;
and     piston means reciprocatably mounted within said filling
sleeve for compacting said filling material within said
cartridge.

## Fig.1a.

## Fig.1b.

## Fig.2a.

## Fig.3a.

## Fig.2b.

## Fig.3b.

*Fig . 3a!.*

16

*Fig.3b!*

15

*Fig.4a.* 20 — 18 — 20

19

*Fig .4b.*

19 — 18

Fig. 5.

Fig. 6a.

Fig. 6b.

54

50

46

51

36